# EUROPÄISCHE PATENTSCHRIFT

(11) **EP 1 179 535 B1**
(45) Veröffentlichungstag und Bekanntmachung des Hinweises auf die Patenterteilung: **22.09.2004**
(21) Anmeldenummer: 01118217.7
(22) Anmeldetag: 28.07.2001
(51) Int. Cl.: C07J 9/00

(54) **Verfahren zur Gewinnung von Sterinen**
Process for the isolation of sterines
Procédé pour l'extraction des sterines

(30) Priorität: 07.08.2000 DE 10038456
(43) Veröffentlichungstag der Anmeldung: 13.02.2002
(73) Patentinhaber: Cognis Deutschland GmbH & Co. KG, 40589 Düsseldorf (DE)
(72) Erfinder: Gutsche, Bernhard, Dr., 40724 Hilden (DE); Bonakdar, Mehdi, Dr., 40764 Langenfeld (DE); Wollmann, Gerhard, Dr., 40723 Hilden (DE); Schwarzer, Jörg, Dr., 40723 Hilden (DE)

(56) Entgegenhaltungen:
- WO-A-94/05650
- DE-C- 19 916 034

## Beschreibung

### Gebiet der Erfindung

Die Erfindung befindet sich auf dem Gebiet der Steringewinnung und betrifft ein Verfahren zur Herstellung von Sterinen aus Rückständen der Destillation von umgeesterten Ölen.

### Stand der Technik

Die Gewinnung von Sterinen aus Destillaten, die bei der Entsäuerung von pflanzlichen Ölen anfallen, oder Destillationsrückständen, die bei der Methylesterproduktion, insbesondere aus der Pflanzenmethylesterherstellung für das Einsatzgebiet "Biodiesel" anfallen, sind in der Patentliteratur bereits verschiedentlich beschrieben worden. Stellvertretend seien hier die Druckschriften **EP-A2 0610742** (Hoffmann-LaRoche), **GB-A1 2145079** (Nisshin Oil Mills Japan) und **EP-A1 0333472** (Palm Oil Research and Development Board) genannt.

Aus dem Europäischen Patent **EP-B1 0656894** (Henkel) ist ein Verfahren zur Herstellung von Sterinen bekannt, bei dem man einen Rückstand aus der Methylesterdestillation, der im wesentlichen aus Glyceriden, Sterinen, Sterinestern und Tocopherolen besteht, in Gegenwart alkalischer Katalysatoren mit Methanol umestert. Die Ausbeute an Sterinen lässt jedoch zu wünschen übrig. Sie wird verbessert in einem Verfahren, das die **DE 19916034** beschreibt.
Die Ausnutzung und Aufwertung von Rückständen aus der Gewinnung von Fetten und Ölen und deren Bestandteilen schließt jedoch häufig aufwendige Verfahren mit Anwendung umweltschädlicher Lösungsmittel ein. In der Patentschrift **US 4148810** werden leichtflüchtige Sterine aus den Destillationsrückständen der Fett- und Ölgewinnung und Aufarbeitung durch Umesterung mit anschließender Lösung in aprotischen, organischen Lösungsmitteln isoliert.

Demzufolge hat die Aufgabe der vorliegenden Erfindung darin bestanden, Sterine in hohen Ausbeuten und in hoher Reinheit durch ein wirtschaftliches Verfahren unter Vermeidung toxikologisch und ökologisch bedenklicher Lösungsmittel herzustellen und dabei Rückstände der Destillation von umgeesterten Ölen ökonomischer auszunutzen.

### Beschreibung der Erfindung

Um Sterine in reiner Form gewinnen zu können, ist es erforderlich, sie aus dem veresterten in den freien Zustand zu versetzen. Anderenfalls lassen sie sich sehr schwer von den Begleitkomponenten abtrennen. Die Umsetzung zu freien Sterinen kann beispielsweise durch Hydrolyse, Verseifung oder Umesterung erfolgen. Die vorliegende Erfindungsmeldung basiert ausschließlich auf der Verfahrensvariante der Umesterung.

Gegenstand der Erfindung ist ein Verfahren zur Gewinnung von Sterinen aus Rückständen der Destillation von umgeesterten Ölen, dadurch gekennzeichnet, dass man
a) die im Gemisch enthaltenen Partialglyceride bei Temperaturen von 115 bis 145 °C und einem Druck von 2 bis 10 bar über 5 - 20 Minuten mit Methanol in Gegenwart eines basischen Katalysators umestert,
b) nach der Umesterung überschüssiges Methanol aus dem Reaktionsgemisch abdestilliert,
c) den Umesterungskatalysator sowie das gegebenenfalls enthaltene Glycerin abtrennt,
d) den Fettsäurealkylester aus dem Gemisch abdestilliert und
e) die im Sumpf enthaltenen Sterinester und restlichen Partialglyceride durch eine weitere Umesterung bei Temperaturen von 90 bis 145 °C und einem Druck von 2 bis 10 bar über 4 - 8 Stunden in freie Sterine und Fettsäureester überführt.

Es wurde gefunden, dass durch die Kombination von zwei getrennten Umesterungschritten ein Verfahren zur Gewinnung von Sterinen wirtschaftlicher und umweltschonender gestaltet werden kann.
Dabei werden in einem ersten Umesterungsschritt die Mono-, Di- und Triglyceride mit Methanol in Gegenwart eines basischen Katalysators umgesetzt. Unter den milden Bedingungen bleiben die Sterinester überwiegend noch gebunden, und es bilden sich nur wenig freie Sterine (< 1 Gew.%). Nach Abtrennung des überschüssigen Alkohols, Umesterungskatalysators und Glycerins erfolgt eine Destillation der Fettsäureester, die die Aufkonzentrierung der Sterinester im Sumpf zur Folge hat. Diese werden jetzt in einem zweiten Umesterungsschritt unter härteren Bedingungen in freie Sterine gespalten. Dadurch, dass bei diesem Umesterungsschritt bereits die Begleitstoffe abgetrennt sind und die Sterinester in aufkonzentrierter Form vorliegen, können die freien Sterine unter wesentlich ökonomischeren Bedingungen gewonnen werden. Die erste Umesterung verläuft sehr schnell und zeitsparend. Deshalb kann als Reaktor ein einfacher Rohrreaktor eingesetzt werden. Durch die reduzierte Menge an Einsatzprodukten kommt man dann bei der zweiten Umesterung mit einem relativ kleinen Rührreaktor aus. Des weiteren lässt sich die Sterinausbeute dadurch erhöhen, dass während der Kristallisation ein Teil der Mutterlauge nach der Filtration der Kristallsuspension in den Kristallisationsprozess zurückgeführt wird.

Das Verfahren ist für unterschiedliche Ausgangsgemische anwendbar und arbeitet ohne toxikologisch und ökologisch bedenkliche Lösungsmittel. Es werden gute Ausbeuten in hoher Qualität erreicht. Die bessere Ausnutzung der Destillationsrückstände führt zu einem wirtschaftlich und ökologischen Verfahren, das im technischem Maßstab einfach durchführbar ist.

### Rückstände der Destillation von umgeesterten Ölen

Als Rohstoffe für die Gewinnung von Sterinen werden Rückstände der Destillation von umgeesterten, insbesondere nicht raffinierten Ölen mit einer Restsäurezahl kleiner 2 eingesetzt. Dies sind vorzugsweise Rückstände aus Kokosöl, aus Palmkernöl, aus Palmöl, aus Sonnenblumenöl und aus Rapsöl mit Säurezahlen von 0 - 6 und enthalten Mischungen aus Di- und Triglyceriden, Methylestern, Sterinestem und Wachsestern, vorzugsweise 35 - 40 Gew.% Triglyceride, 10 - 20 Gew. % Diglyceride, 20 - 25 Gew. % Fettsäuremethylester, 10 - 12 Gew. % Sterinester, 3 - 4 Gew.% Wachsester sowie geringe Mengen freier Sterine und Monoglyceride.

### Erster Umesterungsschritt

### Umesterung der Partialglyceride (a)

Im Verfahrensschritt "Umesterung der Partialglyceride" werden erfindungsgemäß nur die Mono-, Di- und Triglyceride mit Methanol zu Fettsäureestern umgesetzt. Dabei bleiben die Sterinester im Wesentlichen noch gebunden. Es bilden sich nur wenig freie Sterine.

Die zugesetzte Methanolmenge beträgt 5 - 40 Gew.%, vorzugsweise 10 - 20 Gew.% des Rückstandes der Destillation von umgeesterten Ölen.

Vorzugsweise läuft die Reaktion bei 115 - 145°C, besonders bevorzugt 120 -130 °C innerhalb von 5 - 20 min, besonders bevorzugt 8 bis 15 min. ab. Bei den genannten Temperaturen stellt sich ein Druck von 2 - 10 bar ein. Unter diesen Bedingungen, die einer Niederdruckumesterung entsprechen, ist der Zusatz eines Katalysators notwendig. Als Katalysator sind alle Umesterungskatalysatoren geeignet. Vorzugsweise wird hier 30%ige methanolische Natrium-Methylat-Lösung in einer Menge von 0,5 - 1,8 Gew.%, besonders bevorzugt 1,0 - 1,5 Gew. % des Rückstandes der Destillation von umgeesterten Ölen eingesetzt. Als Reaktor werden Batchrührautoklaven als auch kontinuierliche Reaktoren wie z. B. turbulent durchströmte Rohrreaktoren eingesetzt.

Alternativ zur Niederdruckumesterung kann auch eine Druckumesterung durchgeführt werden. Hier läuft die Reaktion vorzugsweise bei 220 - 260°C und einem Druck von 20 - 80 bar ab. Bei Rückständen, die bei der Destillation von unter hohen Drücken umgeesterten Ölen anfallen, ist der Zusatz eines Katalysators nicht notwendig, da der Katalysator, meist Seifen von zweiwertigen Metallen wie Mn, Zn, oder Ca, bereits in hohem Überschuss enthalten ist. Die Druckumesterung bietet sich dann an, wenn die Rückstände der Destillation von umgeesterten Ölen Säurezahlen von über 1 und insbesondere über 5 haben. Der Vorteil der Druckumesterung schlägt insbesondere dann zu Buche, wenn durch diesen Prozess die Säurezahl auf unter 5 und insbesondere auf unter 1 herabgesetzt wird.

### Aufarbeitungsschritte zwischen dem ersten und zweiten Umesterungsschritt

### Flash des Überschuss-Alkohols (b)

Im Verfahrensschritt "Flash des Überschuss-Alkohols" wird das heiße Reaktionsgemisch der "Umesterung der Partialglyceride" in eine Vorlage entspannt. Dabei destillieren 55 - 85 % des überschüssigen Methanols ab. Das System kühlt sich deutlich ab, bei Einsatz von Methanol auf 75 - 85 °C. Der noch im Reaktionsprodukt verbleibende Restalkohol wird vorzugsweise nicht mehr abdestilliert. Er dient als Lösungsvermittler bei der nachfolgenden Stufe.

### Katalysatorfällung und -abtrennung (c)

In den Rückständen der Destillation von unter Druck umgeesterten Ölen sind noch Katalysatoren enthalten. Dies sind vorzugsweise Zn-Seifen (2000 - 3500 ppm), jedoch kommen auch andere Seifen in Frage. Daneben lassen sich in den Destillationsrückständen auch zahlreiche andere Metalle, wie Fe, Al oder Na mit Konzentrationen von bis zu 300 ppm, sowie Schwermetalle wie Pb, Cr oder Ni mit Konzentrationen von bis zu 20 ppm finden. Nichtmetalle wie P, Si oder S sind mit Konzentrationen von bis zu 300 ppm zugegen.

Die Katalysatorseifen sowie die anderen Metallverbindungen sind im Reaktionsgemisch der *"Umesterung der Partialglyceride*" löslich. Um sie abtrennen zu können, werden sie erfindungsgemäß mit Säuren in unlösliche Verbindungen umgewandelt und gefällt. Als Säuren werden vorzugsweise wässrige Lösungen von Citronensäure oder Phosphorsäure eingesetzt. Die Menge der eingesetzten Säure beträgt vorzugsweise die einfache bis doppelte molare Menge der Metallkonzentration. Durch die Säurezugabe wird gleichzeitig das bei der Niederdruckumesterung der Partialglyceride eingesetzte Na-Methylat neutralisiert.

Nach dem Fällen wird der ausgefallene, metallhaltige Schlamm abgetrennt. Vorzugsweise wird er zentrifugiert. Die Phasentrennung verbessert sich, wenn beim Verfahrensschritt *"Flash des Überschuss-Alkohols*" 15-30% des Überschuss-Alkohols noch im Produkt verbleibt.

Alternativ werden die gefällten Metalle adsorbiert. Als Adsorber eignen sich amorphe, mit organischen Säuren beladene Silicagele, wie z. B. Trisyl-Typen der Fa. Grace. Im Falle einer adsorptiven Metallabtrennung kann in der davor geschalteten Verfahrensstufe "*Flash des Überschuss-Alkohols"* der gesamte Alkohol abgetrennt werden.

Bei beiden Alternativverfahren werden Rest-Metallgehalte von unter 1 ppm erreicht.

Das katalysatorfreie Produkt enthält noch Überschuss-Alkohol und freies Glycerin. Damit es bei der nächsten Stufe der *"Destillation der Fettsäureester*" nicht zur Rückreaktion kommt, werden aus dem katalysatorfreien Produkt das freie Glycerin und der Rest-Alkohol durch Dekantieren abgetrennt sowie falls erforderlich mit Wasser ausgewaschen. Das Produkt wird anschließend getrocknet.

### Destillation der Fettsäureester (d)

Zur Anreicherung der Sterinester werden die Fettsäureester abdestilliert. Dies kann beispielsweise in einem Dünnschichtverdampfer geschehen. Methylester werden vorzugsweise bei 170 - 200°C und Drücken von 1 - 5 mbar destilliert. Erfindungsgemäß sind in dem Verfahrensschritt *"Umesterung der Partialglyceride*" im Wesentlichen nur die Partialglyceride umgeestert worden. Da die Sterine noch als Sterinester vorliegen, sind sie höhersiedend und werden bei der Destillation der Fettsäureester nicht abdestilliert. Sie verbleiben erfindungsgemäß vollständig als angereichertes Wertprodukt im Sumpf. Zudem kann eine Abtrennung anderer leicht siedender Komponenten erfolgen.

Erfindungsgemäß werden mit den Fettsäureestern auch Wachsester abdestilliert und anschließend durch Winterisierung von den Fettsäureestern abgetrennt. Auf diese Weise erhält man sterinfreie Methyl- bzw. Ethylester mit einer Reinheit von über 97%.

### Zweiter Umesterungsschritt

### Umesterung der Sterinester (e)

Im Sumpfprodukt der Fettsäureester-Destillation sind die Sterinester auf über 40 % angereichert. Sie werden durch Umesterung mit Methanol, in Gegenwart eines Katalysators in freie Sterine umgesetzt. Da die Umesterung von Sterinestem unter extremeren Bedingungen ablaufen muss als die Umesterung von Partialglyceriden, sind höhere Alkohol- und Katalysatormengen sowie längere Reaktionszeiten nötig.

Die zugesetzte Alkoholmenge beträgt 40 - 80 Gew.%, vorzugsweise 50 - 60 Gew. % des Sumpfproduktes der Fettsäureester-Destillation. Bei Methanol als Umesterungsreagens wird vorzugsweise 40 - 60 Gew.% des Sumpfproduktes der Fettsäureester-Destillation eingesetzt. Als Katalysator sind auch hier alle Umesterungskatalysatoren geeignet.

Vorzugsweise läuft die Reaktion bei 90 - 145°C, besonders bevorzugt 120 - 130°C und einem Druck von 2 - 10 bar innerhalb von 4 - 10 h, vorzugsweise 5 - 8 h ab. Als Katalysator sind alle Niederdruck-Umesterungskatalysatoren geeignet. Vorzugsweise wird 30%ige methanolische Natrium-Methylat-Lösung in einer Menge von 1,8 - 6 Gew. %, vorzugsweise 2 - 4 Gew. % des Sumpfproduktes der Fettsäureester-Destillation eingesetzt. Als Reaktor können beispielsweise Batchrührautoklaven eingesetzt werden.

Alternativ zur Niederdruck-Umesterung kann auch hier eine Druckumesterung durchgeführt werden. Die Reaktion läuft vorzugsweise bei 200 - 260°C und einem Druck von 20 - 80 bar innerhalb von 4 - 8 h ab. Als Katalysator sind alle Hochtemperatur-Umesterungskatalysatoren geeignet. Vorzugsweise werden Zn- oder Ca-Seife eingesetzt.

Die generierten freien Sterine können nun durch Kristallisation aufgereinigt werden. Die Rückstände der Destillation von umgeesterten Ölen, die im erfindungsgemäßen Verfahren als Rohstoffe eingesetzt werden, enthalten jedoch Verunreinigungen, die im Verlauf des hier beschriebenen Prozesses im Produkt noch weiter angereichert werden und den Kristallisationsprozess beeinträchtigen. In diesem Fall können erfindungsgemäß fakultativ weitere Verfahrensschritte wie Flash des Überschuss-Alkohols, Katalysatorabtrennung und Glycerinabtrennung durchgeführt werden, die im Folgenden beschrieben werden.

### Nachgeschaltete Verfahren vor der Kristallisation der Sterine

### Flash des Überschuss-Alkohols (II)

Im Verfahrensschritt *"Flash des Überschuss-Alkohols (II)*" wird das heiße Reaktionsgemisch der *"Umesterung der Sterinester*" in eine Vorlage entspannt. Dabei destillieren 55 - 85 % des Überschuss-Alkohols ab. Das System kühlt sich deutlich ab, bei Einsatz von Methanol auf 75 - 85°C. Der noch im Reaktionsprodukt verbleibende Restalkohol wird vorzugsweise nicht mehr abdestilliert. Er dient als Lösungsvermittler bei der nachfolgenden Stufe.

### Katalysatorabtrennung (II)

Der bei der Umesterung der Sterinester eingesetzte Katalysator ist im Reaktionsgemisch löslich. Um ihn abtrennen zu können, wird er, wie in EP 0656894 B1 beschrieben, mit Säuren in eine unlösliche Verbindung umgewandelt und gefällt.
Nach dem Fällen wird das ausgefallene Salz abgetrennt, Um eine Trennung der organischen von der wässrigen Phase zu realisieren, wird der Mischung erfindungsgemäß 30 - 200 Gew.%, bevorzugt 50-100 Gew.% Fettsäuremethylester, bezogen auf die bei der Umesterung der Sterinester eingesetzte Produktmenge, zugesetzt.

Wird die Umesterung der Sterinester unter Druck und Einsatz von Metallseifen als Katalysator durchgeführt, so kann das gefällte Metall alternativ adsorbiert werden. In diesem Fall kann in der davor geschalteten Verfahrensstufe *"Flash des Überschuss-Alkohols (II)"* der gesamte Alkohol abgetrennt werden. Als Adsorbens eignen sich amorphe, mit organischen Säuren beladene Silicagele, wie z. B. Trisyl-Typen der Fa. Grace.

### Kristallisation der Sterine

Im Folgenden können die freien Sterine durch Kristallisation aufgereinigt werden. Typischerweise ist für eine erfolgreiche Kristallisation eine Konzentration an freien Sterinen von mindestens 20 - 25% erforderlich. Nach dem erfindungsgemäßen Verfahren sind Sterinkonzentrationen von über 40 % realisierbar. Sollte die Konzentration dennoch einen Wert unterschreiten, der eine vernünftige Kristallisation nicht zulässt, so wird die Konzentration dadurch erhöht, dass die im Verfahrensschritt *"Umesterung der Sterinester*" generierten Fettsäureester abdestilliert werden. Hierzu wird analog *"Destillation der Fettsäureester*" vorgegangen.

Wurde die Umesterung der Sterinester unter Druck durchgeführt und die gefällten Metallseifen anschließend adsorptiv entfernt, so wird nun Fettsäuremethylester (FME) als Lösungsmittel zugesetzt. Die FME-Menge beträgt in diesem Fall ebenfalls 30 - 200 Gew.%, bevorzugt 50 - 100 Gew.% bezogen auf die bei der Umesterung der Sterinester eingesetzte Produktmenge.

Bei der Kristallisation wird die Mischung erfindungsgemäß zunächst auf 40 - 120°C, bevorzugt jedoch auf 50 - 90°C aufgeheizt und anschließend auf 20°C abgekühlt. Die Abkühlung kann sowohl unter Rühren als auch ohne Rühren stattfinden. Bei Sterinkonzentrationen von über 25% kann durch Rühren ein Erstarren der gesamten Suspension vermieden werden.

Um die Sterinausbeute zu erhöhen, wird erfindungsgemäß ein Teil der Mutterlauge nach der Filtration der Kristallsuspension, zum Beispiel in den Kristallisationsprozess zurückgeführt. Dabei wird der Rücklaufstrom im Verfahrensschritt *"Katalysatorabtrennung (II)*" zusammen mit den Fettsäureestern dem System zugeführt. Eine weitere Alternative der Rückführung der Mutterlauge stellt die Einführung der Mutterlauge in die erste (a) oder zweite (e) Umesterungsstufe dar.

Das Rücklaufverhältnis der Mutterlauge ist sehr stark vom Einsatzmaterial und somit von der Zusammensetzung der Mutterlauge abhängig. Es kann im Bereich 0,1 - 5,0 liegen. Vorzugsweise wird ein Rücklaufverhältnis von 0,2 - 3,0 eingestellt.

### Kristallisatwäsche

Die erzeugten Kristalle werden mit geeigneten Lösungsmitteln gewaschen und anschließend getrocknet. Man erhält sehr helle Sterinkristalle mit einer Reinheit von über 95%.

### Beispiele

### Beispiel 1:

2,5 kg Rückstand aus der Destillation von umgeestertem Palmkernöl mit einer Restsäurezahl von 3,5 werden mit 375 g (=15%) Methanol in Gegenwart von 37,5 g (=1,5%) 30%igem Na-Methylat bei 122°C umgeestert. Es stellt sich ein Druck von 5 bar ein. Nach Ablauf von 8 min wird die Reaktionsmischung in einen Glaskolben abgelassen, in dem 58 g (=2,3%) 50%ige Citronensäurelösung vorgelegt waren. Dabei werden 80% des Überschuss-Methanols abgeflasht und gleichzeitig der Katalysator neutralisiert. Die Mischung kühlt sich auf 75°C ab.

Nach 15 min Rühren werden zur Reaktionsmischung 250 g Wasser zugegeben und weitere 60 min bei 75°C gerührt. Danach wird abgekühlt und die wässrige Phase abgelassen. Die organische Phase wird zweimal mit jeweils 250 g Wasser gewaschen.

Zur Abtrennung der Methylester wird das Produkt im Dünnschichtverdampfer bei 180°C und 3 mbar destilliert. Der Feedzulauf erfolgt bei 90°C. Die Kondensatortemperatur beträgt 50°C. Bei einem Durchsatz von 150 g/min wird ein Verhältnis Destillat/Sumpf von 75:25 erzielt. Die Methylesterausbeute beträgt somit 70% bezogen auf den Rückstand aus der Destillation von umgeestertem Palmkernöl.

130 g des Sumpfproduktes der ME-Destillation werden durch Zusatz von 65 g (=50%) Methanol und 2,6 g (=2,0%) Na-Methylat bei 120°C umgeestert. Nach 5 h wird durch Zugabe von 4,0 g (=3,1%) 50%ige Citronensäure die Reaktion gestoppt und das Überschuss-Methanol abgeflasht. Die Mischung kühlt sich auf 75°C ab.

Nach 15 min Rühren werden zur Reaktionsmischung 13 g Wasser zugegeben und nach weiteren 30 min Rührzeit bei 75°C 110 g Fettsäuremethylester zugesetzt, um eine Phasentrennung zu ermöglichen. Diese erfolgt bei 60°C. Nach Abtrennung der wässrigen Phase wird die organische Phase mit 39 g Wasser gewaschen.
a) Die organische Phase wird auf 65°C erwärmt und anschließend Rührer und Heizung ausgeschaltet. Nach 60 min kühlt sich die Mischung auf 25°C ab und die maximal erzielbare Kristallmenge wird erhalten.
b) Die organische Phase wird auf 65°C erwärmt und anschließend in einen unbeheizten Behälter gefüllt. Nach 20 min kühlt sich die Mischung auf unter 30°C ab und die maximal erzielbare Kristallmenge wird erhalten.
c) Die organische Phase wird auf 65°C erwärmt und unter Rühren mit einer Geschwindigkeit von 0,5°C/min auf 20°C heruntergekühlt. Es werden Sterinkristalle erhalten, die jedoch in der Menge deutlich geringer sind als bei den Beispielen a) und b).

Die in den Beispielen a) und b) erhaltenen Kristalle werden mit geeigneten Lösungsmitteln gewaschen. Nach Trocknung erhält man 15,5 g Sterine, entsprechend einer Ausbeute von 42,7% bezogen auf den gesamten Steringehalt im Rückstand aus der Destillation von umgeestertem Palmkernöl. Die Sterinkonzentration in Endprodukt beträgt über 95%.

### Beispiel 2:

Der im Beispiel 1 geschilderte Vorgang wird bis einschließlich Umesterung der Sterinester wiederholt. Bei der anschließenden Wäsche wird zur Unterstützung der Phasentrennung zusätzlich zu den 110 g Fettsäuremethylester auch 20% der Mutterlauge, die im Beispiel 1 b) erhalten wurden, zur Mischung gegeben. Alle weiteren Schritte erfolgen analog Beispiel 1, wobei die Kristallisation nach dem Beispiel 1 b) durchgeführt wird.

Durch die Rückführung von 20% der Mutterlauge kann die Sterinausbeute auf 19 g und somit 52,3% bezogen auf den gesamten Steringehalt im Rückstand aus der Destillation von umgeestertem Palmkemöl erhöht werden. Die Sterinkonzentration im Endprodukt beträgt über 95%.

## Patentansprüche

1. Verfahren zur Gewinnung von Sterinen aus Rückständen der Destillation von umgeesterten Ölen, **dadurch gekennzeichnet, dass** man
a) die im Gemisch enthaltenen Partialglyceride bei Temperaturen von 115 bis 145 °C und einem Druck von 2 bis 10 bar über 5 - 20 Minuten mit Methanol in Gegenwart eines basischen Katalysators umestert,
b) nach der Umesterung überschüssiges Methanol aus dem Reaktionsgemisch abdestilliert,
c) den Umesterungskatalysator sowie das enthaltene Glycerin abtrennt,
d) den Fettsäurealkylester aus dem Gemisch abdestilliert und
e) die im Sumpf enthaltenen Sterinester und restlichen Partialglyceride durch eine weitere Umesterung bei Temperaturen von 90 bis 145 °C und einem Druck von 2 bis 10 bar über 4 - 8 Stunden in freie Sterine und Fettsäureester überführt.

2. Verfahren nach Anspruch 1, **dadurch gekennzeichnet, dass** in der ersten Umesterung a) eine Katalysatormenge von 0,5 - 1,8 Gew. % und eine Methanolmenge von 5 bis 40 Gew. % bezogen auf die Menge des umzuesternden Rückstandes eingesetzt wird.

3. Verfahren nach den Ansprüchen 1 und 2, **dadurch gekennzeichnet, dass** in der zweiten Umesterung e) eine Katalysatormenge von 1,8 - 6 Gew. % und eine Methanolmenge von 40 bis 80 Gew. % bezogen auf die Menge des umzuesternden Sumpfproduktes eingesetzt wird.

4. Verfahren nach den Ansprüchen 1 bis 3, **dadurch gekennzeichnet, dass** man die gewonnenen freien Sterine durch nachfolgende Kristallisation und Wäschen aufreinigt.

5. Verfahren nach den Ansprüchen 1 bis 4, **dadurch gekennzeichnet, dass** man die gewonnenen freien Sterine durch nachfolgende Kristallisation aufreinigt, bei der ein Teil der Mutterlauge nach Filtration der Kristallsuspension als Rücklauf in den Kristallisationsprozess zurückgeführt wird.

6. Verfahren nach den Ansprüchen 1 bis 5, **dadurch gekennzeichnet, dass** man Destillationsrückstände von umgeesterten Ölen pflanzlicher Herkunft einsetzt.

## Claims

1. A process for the production of sterols from residues of the distillation of transesterified oils, **characterized in that**
a) the partial glycerides present in the mixture are transesterified with methanol in the presence of a basic catalyst for 5 to 20 minutes at temperatures of 115 to 145°C and under a pressure of 2 to 10 bar,
b) the excess methanol is distilled off from the reaction mixture after the transesterification,
c) the transesterification catalyst and the glycerol present, if any, are removed,
d) the fatty acid alkyl ester is distilled off from the mixture and
e) the sterol esters and residual partial glycerides present in the bottom product are converted into free sterols and fatty acid esters by further transesterification for 4 to 8 hours at temperatures of 90 to 145°C and under a pressure of 2 to 10 bar.

2. A process as claimed in claim 1, **characterized in that** a quantity of catalyst of 0.5 to 1.8% by weight and a quantity of methanol of 5 to 40% by weight, based on the amount of residue to be transesterified, are used in the first transesterification a).

3. A process as claimed in claims 1 and 2, **characterized in that** a quantity of catalyst of 1.8 to 6% by weight and a quantity of methanol of 40 to 80% by weight, based on the amount of bottom product to be transesterified, are used in the second transesterification e).

4. A process as claimed in claims 1 to 3, **characterized in that** the free sterols obtained are purified by subsequent crystallization and washing.

5. A process as claimed in claims 1 to 4, **characterized in that** the free sterols obtained are purified by subsequent crystallization in which part of the mother liquor is recycled to the crystallization process after filtration of the crystal suspension.

6. A process as claimed in claims 1 to 5, **characterized in that** distillation residues of transesterified oils of vegetable origin are used.

## Revendications

1. Procédé pour l'obtention de stérols à partir de résidus de distillation d'huiles transestérifiées,
**caractérisé en ce qu'**
a) on transestérifie les glycérides partiels présents dans le mélange avec du méthanol en présence d'un catalyseur basique à des températures comprises entre 115 et 145 °C et à une pression comprise entre 2 et 10 bar pendant 5 à 20 minutes,
b) on élimine l'excès de méthanol du mélange réactionnel par distillation après la transestérification,
c) on sépare le catalyseur de transestérification ainsi que le glycérol présent,
d) on élimine l'ester alkylique d'acide gras du mélange par distillation et
e) on transforme les esters de stérol et les glycérides partiels résiduels présents dans le résidu de distillation en stérols libres et esters d'acide gras par l'intermédiaire d'une autre transestérification à des températures comprises entre 90 et 145 °C et à une pression comprise entre 2 et 10 bar pendant 4 à 8 heures.

2. Procédé selon la revendication 1,
**caractérisé en ce que**
dans la première transestérification a), on utilise une quantité de catalyseur comprise entre 0,5 et 1,8 % en poids et une quantité de méthanol comprise entre 5 et 40 % en poids, par rapport à la quantité du résidu à transestérifier.

3. Procédé selon les revendications 1 et 2,
**caractérisé en ce que**
dans la seconde transestérification e), on utilise une quantité de catalyseur comprise entre 1,8 et 6 % en poids et une quantité de méthanol comprise entre 40 et 80 % en poids, par rapport à la quantité du produit résiduel de distillation à transestérifier.

4. Procédé selon les revendications 1 à 3,
**caractérisé en ce qu'**
on purifie les stérols libres obtenus par cristallisation subséquente et lavages.

5. Procédé selon les revendications 1 à 4,
**caractérisé en ce qu'**
on purifie les stérols libres obtenus par cristallisation subséquente en recyclant une partie de la solution mère, après filtration de la suspension cristalline, comme reflux dans le processus de cristallisation.

6. Procédé selon les revendications 1 à 5,
**caractérisé en ce qu'**
on utilise des résidus de distillation d'huiles transestérifiées d'origine végétale.
